# EUROPÄISCHE PATENTSCHRIFT

(11) **EP 1 539 276 B1**
(45) Veröffentlichungstag und Bekanntmachung des Hinweises auf die Patenterteilung: **17.05.2006**
(21) Anmeldenummer: 03797300.5
(22) Anmeldetag: 12.09.2003
(51) Int. Cl.: A61M 5/158

(54) **KATHETERKOPF MIT KATHETERABGANG IN DISKRETEN DREHPOSITIONEN**
CATHETER HEAD WITH CATHETER DRAIN IN DISCRETE ROTATIONAL POSITIONS
TETE DE CATHETER AVEC SORTIE DE CATHETER EN POSITIONS DE PIVOTEMENT DISCRETES

(30) Priorität: 12.09.2002 DE 10242419
(43) Veröffentlichungstag der Anmeldung: 15.06.2005
(73) Patentinhaber: Disetronic Licensing AG, 3401 Burgdorf (CH)
(72) Erfinder: HORISBERGER, Ronny-Patrick, CH-3400 Burgdorf (CH); WYSS, Martin, CH-3510 Konolfingen (CH)
(74) Vertreter: Schwabe - Sandmair - Marx
(86) Internationale Anmeldenummer: PCT/EP2003/010179
(87) Internationale Veröffentlichungsnummer: WO 2004/026375

(56) Entgegenhaltungen:
- US-A- 5 176 662
- US-A- 5 968 011
- US-A- 6 017 328
- US-A1- 2002 123 724

## Beschreibung

Die Erfindung betrifft einen Katheterkopf zur Einleitung eines Fluids in ein organisches Gewebe, insbesondere einen Katheterkopf zur Verabreichung eines flüssigen Wirkstoffes.

Solche Katheterköpfe werden z.B. in Verbindung mit Infusionsvorrichtungen eingesetzt, um bei einem Patienten, dem dauerhaft oder wiederholt ein Fluid verabreicht wird, das Wechseln eines Teils des Katheterkopfes, d. h. des Kanülengehäuses mit einer in einem Körper eingebrachten Kanüle, oder das Austauschen eines Fluids zu ermöglichen. Ein anderer Teil des Katheterkopfes, das Verbindungselement mit einer Fluidzuführung, wird hierfür von dem Kanülengehäuse gelöst und kann nach dem Auswechseln des Kanülengehäuses zur weiteren Einleitung des Fluids in den Körper auf den neuen am Körper angebrachten Kanülengehäuse aufgesetzt werden. Gleichzeitig kann an das Verbindungselement problemlos ein neuer Fluidbehälter angeschlossen werden. Es ist auch möglich, ein anderes gleichartiges Verbindungselement eines neuen Fluidbehälters auf das Kanülengehäuse aufzusetzen, sodass die Verabreichung des Fluids fortgesetzt werden kann. Ein solcher Katheterkopf kann aber auch verwendet werden, um z.B. Analyseflüssigkeit aus dem Körper eines Patienten zu entnehmen oder die Analyseflüssigkeit in den Körper ein- und wieder auszuleiten.

Im Allgemeinen weist das Känülengehäuse des Katheterkopfes eine Kanüle, die von einer Seite des Kanülengehäuses hervorsteht, und einen Durchgangskanal auf, der durch das Kanülengehäuse verläuft und mit der Kanüle verbunden ist. Das Verbindungselement des Katheterkopfes besitzt eine Fluidzuführung und wird mit dem Kanülengehäuse derart verbunden, dass die Fluidzuführung mit dem Durchgangskanal verbunden wird. Weiter ist eine Führungsvorrichtung vorgesehen, die beim Zusammensetzen des Kanülengehäuses mit dem Verbindungselement das Verbindungselement in die richtige Position auf das Kanülengehäuse führt. Durch eine Befestigungsvorrichtung wird das Verbindungselement fest auf dem Kanülengehäuse fixiert, ist jedoch wieder lösbar.

Ein derartiger Katheterkopf ist z.B. in der DE 299 05 068 bei einer subkutanen Infusionsvorrichtung beschrieben. Bei diesem Katheterkopf wird das Verbindungselement in das Kanülengehäuse eingesteckt, sodass die Fluidzuführung als Verlängerung der Kanüle angeordnet ist. Hierbei ist die Führungsvorrichtung und die Befestigungsvorrichtung achsensymmetrisch in Bezug zur Längsachse der Fluidzuführung ausgebildet. Ein erster Teil der Führungsvorrichtung ist als Hohlraum mit einer kreisförmigen inneren Wand in dem Kanülengehäuse vorgesehen. Ein zweiter Teil der Führungsvorrichtung ist durch eine äußere Wand des Verbindungselements gegeben, die beim Zusammensetzen an der inneren Wand des Hohlraums in dem Kanülengehäuse anliegt. Nach dem Zusammensetzen des Kanülengehäuses und dem Verbindungselement ist daher das Verbindungselement in dem Kanülengehäuse drehbar angeordnet. Es ist ein Verblockungsmechanismus vorgesehen, der das Verbindungselement in Bezug zum Gehäuse in irgendeiner Winkelposition fixiert. In jeder dieser Winkelpositionen bildet jedoch die Fluidzuführung die Verlängerung der Kanüle. Es werden keine unterschiedlichen Winkelpositionen zwischen Kanüle und Fluidzuführung gebildet. Ein solcher Verblockungsmechanismus ist z.B. durch einen an dem Verbindungselement vorgesehenen flexiblen Verschlussarm mit einem außenliegenden Vorsprung vorgesehen. Nach dem Einführen des Verbindungselements in das Kanülengehäuse greift der Vorsprung in eine kreisförmige Nut im Inneren des Kanülengehäuses ein und fixiert auf diese Weise das Verbindungselement mit dem Kanülengehäuse. Zum Lösen der Verblockung wird der flexible Verschlussarm nach innen gebogen und das Verbindungselement kann aus dem Kanülengehäuse herausgezogen werden.

Aus der US 6,017,328 ist ein Katheterkopf bekannt, der ein plattenförmiges Kanülengehäuse mit einer nach unten ragenden Kanüle aufweist. Ein ebenfalls plattenförmiges Verbindungselement wird derart auf das Kanülengehäuse aufgesetzt, dass die Fluidzuführung im wesentlichen senkrecht zur Achse der Kanüle angeordnet ist. Das Befestigungselement weist zwei seitlich verlaufende Arme auf, die zur Mitte des Befestigungselements hin zusammengedrückt werden können. Die Arme weisen an ihrem Ende in Richtung des Kanülengehäuses gerichtete Vorsprünge auf, mit welchen sie in passende Aussparungen an dem Kanülengehäuse fest eingreifen. Durch das Zusammendrücken der Arme kann der Eingriff wieder gelöst werden. Durch die vorbestimmte Position der Aussparungen an dem Kanülengehäuse ist die Winkelstellung des Verbindungselements im Bezug zu dem Kanülengehäuse festgelegt. Die Richtung, in der die Fluidzuführung von dem Kanülengehäuse wegführt, ist daher vorbestimmt.

Bei den Katheterköpfen nach dem Stand der Technik ist es entweder überhaupt nicht möglich, eine Winkeleinstellung zwischen dem Kanülengehäuse, bzw. der Kanüle, und der Fluidzuführung auszuwählen oder aber es ist bereits eine bestimmte Winkelstellung vorgegeben, die nicht variierbar ist. Wird es gewünscht, dass die Fluidzuführung aus einer geänderten Richtung erfolgt, muss daher z.B. ein Zuführungsschlauch mit genügend großer Länge gewählt werden, der eine Biegung in die gewünschte geänderte Richtung ermöglicht ohne den Durchfluss zu stören. Oder aber es muss der gesamte Katheterkopf bei in dem Patienten verbleibender Kanüle gedreht werden, was für den Patienten unangenehm und inakzeptabel ist.

Die Erfindung hat es sich zur Aufgabe gemacht, einen Katheterkopf zur Einleitung eines Fluids in ein organisches Gewebe zu schaffen, dessen Kanülengehäuse und Verbindungselement auf einfache Weise miteinander verbunden werden können und der eine variable Fluidzuführung zu dem Gewebe ermöglicht.

Diese Aufgabe wird erfindungsgemäß nach Anspruch 1 gelöst. Vorteilhafte Ausgestaltungen der Erfindung gehen aus den Unteransprüchen hervor.

Nach der Erfindung weist ein Katheterkopf der vorher beschriebenen Art für die Positionierung des Verbindungselements relativ zu dem Kanülengehäuse um eine Längsachse der Kanüle mehrere wählbare diskrete Drehpositionen auf. Das in einer Drehposition positionierte Verbindungselement wird durch die Befestigungsvorrichtung in der ausgewählten Drehposition an dem Kanülengehäuse lösbar befestigt. Bei einem Katheterkopf ist es auch möglich, die Befestigungsvorrichtung zu lösen, eine andere Positionierung des Verbindungselements relativ zu dem Kanülengehäuse auszuwählen und das Verbindungselement in dieser Drehposition an dem Kanülengehäuse wieder zu befestigen, ohne das Verbindungselement vollständig von dem Kanülengehäuse zu trennen. Vorzugsweise wird die Fluidzuführung beim Befestigen des Verbindungselements an dem Kanülengehäuse winkelig zur Längsachse der Kanüle angeordnet. Mit dem erfindungsgemäßen Katheterkopf ist es möglich, beim Einleiten oder Verabreichen eines Fluids in ein organisches Gewebe eine Fluidzuführung aus unterschiedlichen Richtungen vorzusehen. Dadurch wird der Bewegungsspielraum eines Patienten erweitert und es kann eine optimale Einleitung des Fluids sichergestellt werden.

Bei einer bevorzugten Ausführungsform des erfindungsgemäßen Katheterkopfes ist das Kanülengehäuse im wesentlichen flach, bzw. plattenartig ausgebildet. Vorzugsweise ist es in einer Draufsicht kreisförmig. Von der flächenartigen, beispielsweise der ebenen Seite, die dem Gewebe zugewandt ist, steht die Kanüle im wesentlichen senkrecht hervor. Jedoch ist es auch denkbar die Kanüle in einem Winkel zu der Normalen des Kanülengehäuses anzubringen. Vorzugsweise ist auf dieser Seite die Kanüle im Zentrum des Kanülengehäuses vorgesehen, kann aber auch am Rand des Kanülengehäuses angeordnet werden. Auf der gegenüberliegenden flächigen Seite, die von dem Gewebe abgewandt ist, wird das ebenfalls flächige und vorzugsweise kreisförmige Verbindungselement angeordnet. Die Fluidzuführung ist dabei nach dem Befestigen des Verbindungselements an dem Kanülengehäuse vorzugsweise im wesentlichen senkrecht zur Symmetrieachse des Kanülengehäuses angeordnet, d. h. das einzuleitende Fluid wird innerhalb des Katheterkopfes um ca. 90 Grad umgelenkt. Es kann aber auch je nach den Anforderungen an das Einleiten eines Fluids eine andere Winkelstellung erfüllt werden oder die Fluidzuführung kann in der Verlängerung der Kanüle angeordnet sein. Dabei ist es vorteilhaft, wenn die einzelnen Drehpositionen der Führungsvorrichtung frei wählbar sind, so kann die Fluidzuführung in den Katheterkopf aus einer beliebigen auf die speziellen Anforderungen abgestimmten Richtung erfolgen. Beim Zusammensetzen des Kanülengehäuses und des Verbindungselements kann die Flüssigkeitsverbindung z.B. durch eine Nadel am Ende der Fluidzuführung des Verbindungselements, die in den Durchgangskanal des Kanülengehäuses eingeführt wird, gebildet werden. Es ist aber auch möglich, in dem Aufbau des Katheterkopfes einen gemeinsamen Übergangsbereich vorzusehen, in den sowohl die Fluidzuführung des Verbindungselements als auch der Durchgangskanal des Kanülengehäuses münden.

Vorzugsweise wird zur Ausbildung der verschiedenen wählbaren diskreten Drehpositionen zwischen dem Kanülengehäuse und dem Verbindungselement eine Rastverbindung vorgesehen, wobei die einzelnen Rasteinstellungen den diskreten Drehpositionen entsprechen. Hierfür sind z.B. zwei aufeinander abgestimmte Rastmittel vorgesehen. Ein erstes Rastmittel ist wenigstens auf einem Teilbereich einer ringförmigen Fläche, bzw. einer Ringfläche des Kanülengehäuses angeordnet. Ein zweites Rastmittel ist zumindest auf einem Teilbereich einer Ringfläche des Verbindungselements angeordnet und ist gegen das erste Rastmittel gerichtet. Die Teilbereiche einer Ringfläche entsprechen dabei einer Sektorfläche entlang des Umfangs um das Zentrum, d. h. den Mittelpunkt des kreisförmigen Kanülengehäuses bzw. des Verbindungselements, wodurch die Rastmittel auf Ringsektorflächen ausgebildet sind. Die Ringsektorfläche des ersten Rastmittels auf dem Kanülengehäuse und die Ringsektorfläche des zweiten Rastmittels auf dem Verbindungselement sind einander zugewandt, sodass die Rastmittel beim Zusammensetzen des Katheterkopfes zusammenwirken können. Die Ringsektorflächen der Rastmittel können dabei sowohl in einem Bereich nahe des Zentrums, in einem vom Zentrum entfernt gelegenen Bereich, als auch an einem Außenumfang des Kanülengehäuses, bzw. des Verbindungselements angeordnet sein. Es ist nur darauf zu achten, dass die Anordnung der Ringsektorflächen auf dem Kanülengehäuse und auf dem Verbindungselement aufeinander abgestimmt sind. Beim Aufeinandersetzen des Verbindungselements und des Kanülengehäuses können diese beiden Teile gegeneinander um das Zentrum gedreht werden, bis sie eine gewünschte Drehposition zueinander haben. Durch weiteres Zusammenfügen der beiden Teile wird das Verbindungselement in dieser ausgewählten Stellung entlang der dazu passenden Rasteinstellung auf das Kanülengehäuse geführt. Durch die unterschiedlichen Raststellungen der Rastverbindung werden demnach mehrere wählbare diskrete Drehpositionen des Verbindungselements relativ zu dem Kanülengehäuse vorgesehen.

In einer besonders bevorzugten Ausführungsform werden die ersten und zweiten Rastmittel jeweils durch Vorsprünge wie z.B. Rippen gebildet. Dabei ist es möglich, dass eines der beiden Rastmittel durch mehrere Vorsprünge und das andere Rastmittel nur durch einen Vorsprung gebildet wird. Vorzugsweise weisen jedoch beide Rastmittel mehrere Vorsprünge auf. Die Vorsprünge sind zumindest teilweise in Richtung des Zentrums des Kanülengehäuses, bzw. des Verbindungselements gerichtet, insbesondere sind sie in radialer Ausrichtung auf das Zentrum angeordnet. Beim Positionieren des Verbindungselements auf dem Kanülengehäuse greifen die zweiten Vorsprünge des Verbindungselements zwischen die ersten Vorsprünge des Kanülengehäuses. Dabei können die von dem Ringsektorbereich hervorstehenden Flächen der Vorsprünge schräg zur von diesem Bereich gebildeten Grundfläche angeordnet sein, um ein sanftes Ineinandergleiten der ersten und zweiten Vorsprünge zu ermöglichen. In dieser Ausführungsform bewegen sich die ersten und zweiten Rastmittel im wesentlichen direkt auf einander zu. Es ist jedoch auch möglich, das erste Rastmittel an einer äußeren Umfangsfläche des Kanülengehäuse durch Vorsprünge vorzusehen, die radial nach außen von dem Kanülengehäuse hervorstehen. Hierfür könnten z.B. Zacken am Rand des Kanülengehäuses vorgesehen sein. Als hierzu passende zweite Rastmittel können an dem Verbindungselement am äußeren Rand axial in Richtung des Kanülengehäuses hervorstehende Vorsprünge vorgesehen sein, die zum Positionieren des Verbindungselements in die Zwischenräume der Zacken an dem Kanülengehäuse eingreifen.

Für die vorliegende Erfindung ist es ausreichend, wenn die ersten und zweiten Rastmittel auf Teilbereichen einer Ringfläche angeordnet sind. Die Rastmittel können aber auch auf der gesamten Ringfläche vorgesehen sein. Ferner besteht die Möglichkeit, die Rastmittel des Kanülengehäuses und des Verbindungselements auf einem dieser Teile auf der gesamten Ringfläche und auf dem anderen der Teile nur auf einem Teilbereich der Ringfläche auszubilden. Vorzugsweise sind die ersten Rastmittel des Kanülengehäuses auf der gesamten Umfangsringfläche und die zweiten Rastmittel des Kanülengehäuses nur auf einer Ringsektorfläche ausgebildet. Diese Aufteilung der Rastelemente ermöglicht es, auf dem gesamten Umfang des Katheterkopfes diskrete Drehpositionen vorzusehen, die beim Positionieren des Verbindungselements auf dem Kanülengehäuse frei wählbar sind.

Nach einer bevorzugten erfindungsgemäßen Ausführungsform kann die Befestigungsvorrichtung durch eine lösbare Klemmverbindung zwischen dem Kanülengehäuse und dem Verbindungselement gebildet werden. Durch eine solche Klemmverbindung wird das Verbindungselement auf dem Kanülengehäuse nach dessen Positionierung in einer gewählten Drehposition fixiert. Vorzugsweise wird die Klemmverbindung durch wenigstens einen länglichen Öffnungsschlitz durch das Verbindungselement gebildet. Durch den Öffnungsschlitz wird wenigstens ein Außenbereich in dem Verbindungselement definiert, der in Richtung zum Zentrum beweglich ist. Der Außenbereich ist z. B. durch einen im wesentlichen auf das Zentrum des Verbindungselements gerichteten Druck in dieser Richtung beweglich. Dieser Druck kann z.B. durch Zusammendrücken des Verbindungselements zwischen Daumen und Zeigefinger ausgeübt werden. Beim Nachlassen des Drucks wird der Außenbereich wieder zurückgestellt. Zur Klemmverbindung ist wenigstens ein erstes Klemmelement an dem Kanülengehäuse angeordnet. Wenigstens ein zweites Klemmelement ist an dem durch den Öffnungsschlitz definierten Außenbereich des Verbindungselements angeordnet, sodass er beim Zusammendrücken des Außenbereichs wie dieser beweglich ist. Das zweite Klemmelement ist an einer dem Kanülengehäuse zugewandten Seite auf dem Verbindungselement angeordnet und wirkt mit dem ersten Klemmelement zusammen. Die länglichen Öffnungsschlitze können am Rand des Verbindungselements beginnen und in dessen Innerem enden, wodurch der Außenbereich als eine Art Arm von dem Verbindungselement abgeteilt wird. Der längliche Öffnungsschlitz kann aber auch vollständig im Inneren des Verbindungselements liegen. Es ist nur zu beachten, dass der längliche Verbindungsschlitz zumindest teilweise in Umfangsrichtung verläuft, um einen beweglichen Außenbereich des Verbindungselements zu bilden.

In einer besonders bevorzugten Ausführungsform der vorliegenden Erfindung wird das erste Klemmelement durch einen oder mehrere erste Widerhaken gebildet, die sich zumindest auf einem Teilbereich auf dem Kanülengehäuse in Umfangsrichtung erstreckt und in Richtung des Verbindungselements gerichtet ist. Vorzugsweise ist ein erster Widerhaken vorgesehen, der ringsektorförmig ausgebildet ist und entlang eines Ringsektors auf dem Kanülengehäuse angeordnet ist. Es ist aber auch denkbar, mehrere z. B. rechteckförmige Widerhaken nebeneinander auf dem Ringsektor des Kanülengehäuses anzuordnen. Das zweite Klemmelement wird durch einen oder mehrere zweite Widerhaken gebildet, die zu den ersten Widerhaken komplementär ausgestaltet sind. Vorzugsweise ist ein zweiter Widerhaken vorgesehen, der sich zumindest auf einen Teilbereich an dem Außenbereich des Verbindungselements in Umfangsrichtung erstreckt und in Richtung des Kanülengehäuses gerichtet ist. Vorzugsweise ist auch dieser Teilbereich als Ringsektor ausgebildet. Auch hier sind mehrere nebeneinander auf dem Ringsektor angeordnete zweite Widerhaken denkbar. Ferner können auf dem Kanülengehäuse oder dem Verbindungselement ein einziger länglicher, bzw. ringsektorförmiger Haken und entsprechend auf dem anderen der beiden Teile ein oder mehrere kürzere Haken vorgesehen sein. Es ist vorteilhaft zwei längliche Öffnungsschlitze auf einer in Bezug auf das Zentrum gegenüberliegenden Seite des Verbindungselements vorzusehen, wodurch zwei Außenbereiche mit jeweils wenigstens einem Widerhaken definiert werden. Zum Befestigen des Kanülengehäuses mit dem Verbindungselement können die Außenbereiche des Verbindungselements mit dem Widerhaken zusammengedrückt werden, sodass dieser zweite Widerhaken in einen komplementären ersten Widerhaken am Kanülengehäuse eingreifen kann. Beim Befestigen des Verbindungselements an dem Kathetergehäuse durch die Befestigungsvorrichtung können das erste und zweite Rastmittel der Führungsvorrichtung aneinander gleiten. Sind beispielsweise auf einer Ringsektorfläche des Verbindungselements Vorsprünge als Rastmittel der Führungsvorrichtung vorgesehen, dann können diese beim Eindrücken des Außenbereichs an den komplementären auf dem Kanülengehäuse ausgebildeten Vorsprüngen entlang gleiten, wenn die Außenbereiche zusammengedrückt werden.

Als Klemmverbindung der Befestigungsvorrichtung kann auch eine beliebige andere bekannte Klemmverbindung verwendet werden, wie z.B. eine formschlüssige Steckverbindung mit entsprechenden Klemmelementen an dem Kanülengehäuse und dem Verbindungselement.

Gemäß der vorliegenden Erfindung ist es auch möglich, die Führungsvorrichtung zu einem Teil oder vollständig an der Befestigungsvorrichtung vorzusehen. Hierfür kann z.B. die Funktion der Führungsvorrichtung durch ein Zusammenwirken des ersten und des zweiten Klemmelements der Befestigungsvorrichtung erfüllt werden. Beispielsweise kann an dem ersten Klemmelement zusätzlich ein erstes Rastmittel, wie z.B. Zähne oder Stifte, und an dem zweiten Klemmelement kann zusätzlich ein zweites Rastmittel, wie z.B. komplementäre Zähne oder Löcher für die Stifte, vorgesehen sein. Dadurch kann gleichzeitig mit dem Befestigen des Verbindungselements an dem Kanülengehäuse eine bestimmte Rasteinstellung als diskrete Drehposition ausgewählt werden.

Durch die rotationssymmetrische Anordnung der Elemente der Führungs- und Befestigungsvorrichtung um das Zentrum des Kanülengehäuses bzw. des Verbindungselements kann das Verbindungselement relativ zu dem Kanülengehäuse in eine bestimmte Drehposition gebracht werden, wobei die jeweiligen komplementären ersten und zweiten Rast- und Klemmelemente aufgrund der Drehsymmetrie in jeder Drehposition passend einander gegenüberliegen und zusammen wirken können.

Vorteilhafte Ausführungsbeispiele der Erfindung werden nachfolgend anhand der Zeichnung erläutert. Aus der Zeichnung hervorgehende Aus- und Weiterbildungen der Erfindung sollen als zur Offenbarung der Erfindung gehörend betrachtet werden. In der Zeichnung stellen dar:
Figur 1: eine perspektivische Ansicht mit einem Teilschnitt durch einen Katheterkopf nach einer Ausführungsform der Erfindung und
Figur 2: eine perspektivische Aufsicht auf ein Kanülengehäuse nach einer anderen Ausführungsform der Erfindung.

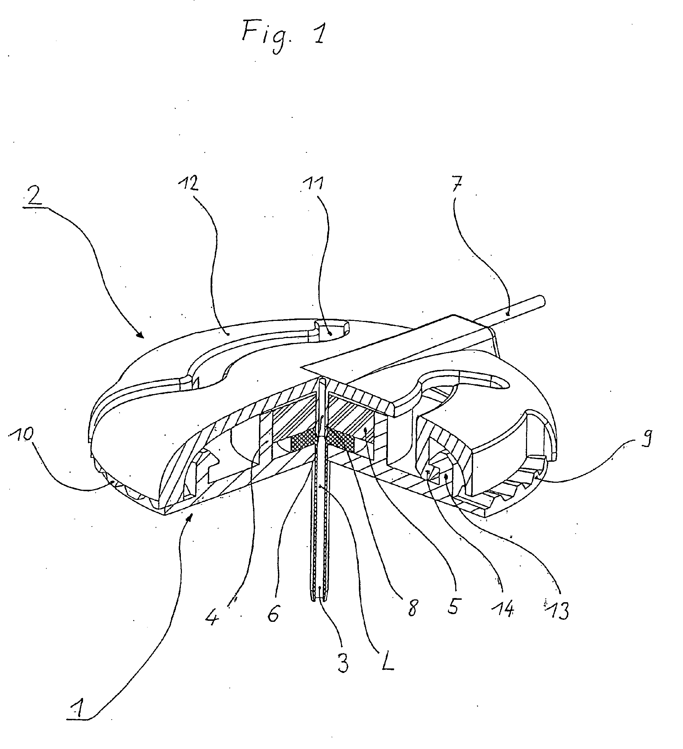

In Figur 1 ist ein Katheterkopf nach der vorliegenden Erfindung mit einem kreisförmigen flachen Kanülengehäuse 1 und einem darauf befestigten kreisförmigen, flachen Verbindungselement 2 dargestellt. Von dem Kanülengehäuse 1 steht eine Kanüle 3 hervor, die in der Zeichnung im wesentlichen senkrecht von dem Kanülengehäuse 1 nach unten gerichtet ist. Die Kanüle 3 kann jedoch in einem beliebigen anderen Winkel von dem Kanülengehäuse 1 hervorstehen und damit in einem beliebigen Winkel in organisches Gewebe eindringen. In dem gezeigten Beispiel ragt eine ringförmig um eine zentrale Mittelöffnung angeordnete Wand 4 nach oben von dem Kanülengehäuse 1. Die Kanüle 3 wird durch die zentrale Mittelöffnung geführt und von einem innerhalb des Umfangs der Wand 4 eingesetzten Einsatzteils 5, d. h. einem Septum, gesichert. Das Septum 5 weist ebenfalls eine zentrale Mittelöffnung auf, die den Durchgangskanal 6 bildet.

Das Verbindungselement 2 weist eine Fluidzuführung 7 auf, die in einen röhrenartigen zentralen Fortsatz 8 mündet, der zentral in der Mitte des Verbindungselements 2 angeordnet ist. Der Fortsatz 8 wird entlang der zentralen Mittelöffnung des Septums 5 geführt, bis er in die Kanüle 3 mündet, wodurch eine Flüssigkeitsverbindung von einem Fluidbehälter (nicht gezeigt) über die Fluidzuführung 7 und die Kanüle 3 in ein organisches Gewebe erzeugt wird. Ist das Verbindungselement 2 erst teilweise in die Mittelöffnung des Septums 5 eingeführt, kann es um die Achse des röhrenförmigen Fortsatzes 8, die gleichzeitig auch die Verlängerung der Längsachse der Kanüle darstellt, relativ zu dem Kanülengehäuse gedreht werden.

In dem in Figur 1 gezeigten Beispiel wird die Führungsvorrichtung gemäß der vorliegenden Erfindung durch erste Vorsprünge 9 an dem Kanülengehäuse 1 und durch zweite Vorsprünge 10 an dem Verbindungselement 2 gebildet. Die Vorsprünge 9 sind auf einer äußeren nach oben weisenden Ringfläche des Kanülengehäuses 1 angeordnet, wobei die ersten Vorsprünge auf dieser Ringfläche radial zum Zentrum des Kanülengehäuses verlaufen. Die zweiten Vorsprünge 10 des Verbindungselements 2 sind an der Umfangsrandfläche des Verbindungselements 2 angeordnet, wobei der Rand des Verbindungselements 2 mit den zweiten Vorsprüngen 10 derart in Richtung des Kanülengehäuses 1 gewölbt ist, dass die zweiten Vorsprünge 10 den ersten Vorsprüngen 9 gegenüber angeordnet sind. Die zweiten Vorsprünge 10 verlaufen daher ebenfalls radial in Richtung auf das Zentrum des Kanülengehäuses 1 bzw. des Verbindungselements 2.

Beim Aufsetzen des Verbindungselements 2 auf das Kanülengehäuse 1, d.h. beim Einführen des Fortsatzes 8 in die Mittelöffnung des Einsatzteils 5, kann das Verbindungselement 2 relativ zu dem Kanülengehäuse 1 soweit gedreht werden, bis die Fluidzuführung 7 in einer gewünschten Richtung von dem Katheterkopf wegführt. Diese ausgewählte Stellung entspricht entweder direkt einer der diskreten Drehpositionen der Führungsvorrichtung oder kann durch eine geringfügige vernachlässigbare Winkelbewegung in eine nahegelegene zu einer diskreten Drehposition passende Stellung gebracht werden. Diese diskrete Drehposition wird durch das Eingreifen der zweiten Vorsprünge 10 an dem Verbindungselement 2 in den in dieser Stellung gegenüberliegenden Zwischenraum zwischen zwei ersten Vorsprüngen 9 auf dem Kanülengehäuse 1 bestimmt. Beim weiteren Zusammenfügen des Verbindungselements 2 und des Kanülengehäuses 1 werden diese beiden Teile durch die ineinander gleitenden Vorsprünge 9 und 10 geführt. Um das Auffinden der diskreten Position und ein sanftes Einführen zu ermöglichen, sind die aufragenden Seiten der Vorsprünge schräg zu ihrer Grundfläche angeordnet. Das Verbindungselement 2 wird so lange in das Kanülengehäuse 1 eingeführt, bis die zweiten Vorsprünge 10 auf der Grundfläche der Zwischenräume der ersten Vorsprünge 9 aufstehen. In diesem Zustand ragt der Fortsatz 8 mit einer gewissen Tiefe in die Kanüle 3 hinein, um die Fluidverbindung sicherzustellen. Wird eine andere Drehposition des Verbindungselements 2 relativ zu dem Kanülengehäuse 1 gewünscht, kann das Verbindungselement 2 um einen kurzen Weg von dem Kanülengehäuse 1 angehoben werden, bis der Eingriff der Vorsprünge 9 und 10 gelöst ist. Dann kann das Verbindungselement 2 wieder relativ zu dem Kanülengehäuse 1 gedreht werden, bis eine gewünschte neue Drehposition erreicht ist.

Durch Zusammenschieben des Verbindungselements 2 und des Kanülengehäuses 1 werden nun die zweiten Vorsprünge 10 des Verbindungselements 2 entsprechend der neuen diskreten Drehposition in den ihnen nun gegenüberliegenden Zwischenraum zwischen den ersten Vorsprüngen 9 des Kanülengehäuses 1 eingeführt. Vorzugsweise verbleibt der Fortsatz 8 beim Anheben des Verbindungselements 2 von dem Kanülengehäuse 1 zur Änderung einer Drehposition zumindest mit seiner Spitze in der Mittelöffnung des Septums 5, vorzugsweise in der Kanüle 3, um trotz der Änderung der Drehposition die Fluidverbindung nicht zu unterbrechen.

Zur Ausbildung der Befestigungsvorrichtung sind in dem in Figur 1 gezeigten Beispiel nach der Erfindung zwei längliche Öffnungsschlitze 11 durch das Verbindungselement 2 angeordnet. Die Schlitze beginnen vom Rand des Verbindungselements 2 und verlaufen zunächst im wesentlichen in Richtung zum Zentrum des Verbindungselements 2, gehen anschließend in einen im wesentlichen in Umfangsrichtung verlaufenden Teil über und enden in einem im wesentlichen wieder radial nach außen verlaufenden Teil vor dem Rand des Verbindungselements 2. Sie sind demnach in einer Art Wellenform ausgebildet. Durch diese Öffnungsschlitze 11 werden zwei Außenbereiche 12 des Verbindungselements 2 definiert. Diese Außenbereiche bilden jeweils eine Art Arme des Verbindungselements 2, die in Richtung des Zentrums des Verbindungselements 2 beweglich sind, wenn vom Rand ein Druck auf diese Außenbereiche ausgeübt wird.

Auf dem Kanülengehäuse 1 ist ein erster Widerhaken 13 als ein erstes Klemmelement der Befestigungsvorrichtung vorgesehen, der nach oben in Richtung des Verbindungselements 2 von dem Kanülengehäuse 1 ragt. Vorzugsweise wird der erste Widerhaken 13 durch eine in Richtung des Zentrums des Kanülengehäuses hervorstehende Nase gebildet und ist ringförmig um das Zentrum des Kanülengehäuses 1 umlaufend auf dem gesamten Umfang des Kanülengehäuses angeordnet. Es ist jedoch auch möglich, den Widerhaken nur auf Umfangssektoren anzuordnen. An dem Verbindungselement 2 sind an den Außenbereichen 12 nach unten in Richtung des Kanülengehäuses 1 ragende zu dem ersten Widerhaken 13 komplementäre zweite Widerhaken 14 als zweite Klemmelemente der Befestigungsvorrichtung angeordnet. Die Widerhaken 14 erstrecken sich in Umfangsrichtung am Rand des in Umfangsrichtung verlaufenden Teils der Öffnungsschlitze 11. Dabei können die zweiten Widerhaken 14 durchgehend entlang des in Umfangsrichtung verlaufenden Teils des Öffnungsschlitzes 11 verlaufen oder unterbrochen sein.

Beim Aufsetzen des Verbindungselements 2 auf das Kanülengehäuse 1 werden die Außenbereiche 12 des Verbindungselements 2 z.B. durch den Daumen und den Zeigefinger zusammengedrückt, sodass sich die zweiten Widerhaken 14 zum Zentrum des Verbindungselements 2 hin bewegen und beim Einführen des Verbindungselements 2 in das Kanülengehäuse 1 an dem ersten Widerhaken 13 des Kanülengehäuses 1 vorbeigeführt werden. Vorzugsweise sind die Außenbereiche 12 soweit nach innen beweglich, dass die zweiten Widerhaken 14 nicht an dem ersten Widerhaken 13 reiben.

Ist eine Drehposition ausgewählt und das Kanülengehäuse 1 und das Verbindungselement 2 in dieser Drehposition vollständig ineinander geführt, wird der Druck auf die Außenbereiche 12 vermindert und die Widerhaken 13 und 14 greifen in einer Klemmverbindung ineinander. Soll eine neue Drehposition ausgewählt werden, werden die Außenbereiche 12 erneut zusammengedrückt, um die Klemmverbindung der Widerhaken 13 und 14 zu lösen, sodass das Verbindungselement 2 zumindest teilweise von dem Kanülengehäuse 1 angehoben werden kann, zumindest soweit, bis die Rastverbindung der Führungsvorrichtung gelöst ist. Das Verbindungselement 2 und das Kanülengehäuse 1 können dann relativ zueinander in eine neue gewünschte Drehposition gedreht werden. Durch vollständiges Zusammenschieben des Kanülengehäuses und des Verbindungselements bei eingedrückten Außenbereichen 12 können wiederum die zweiten Vorsprünge 10 in die zu der neuen Drehposition passenden Zwischenräume zwischen den ersten Vorsprüngen 9 eingreifen. Durch Nachlassen des Drucks auf die Außenbereiche 12 greifen die Widerhaken 13 und 14 ineinander und das Verbindungselement 2 wird auf dem Kanülengehäuse 1 in dieser neuen diskreten Drehposition befestigt.

In dem in Figur 1 gezeigten Beispiel sind die ersten Vorsprünge 9 auf der gesamten Umfangsfläche des Kanülengehäuses 1 vorgesehen, wohingegen die zweiten Vorsprünge 10 des Verbindungselements 2 nur am Rand der zwischen den beweglichen Außenbereichen liegenden Bereiche vorgesehen sind, d.h. am Rand der Außenbereiche sind in dem gezeigten Beispiel keine zweiten Vorsprünge 10 vorgesehen. Dadurch wird das Eingreifen der Klemmelemente 13 und 14 erleichtert. Es wäre jedoch ebenso möglich, auch am Rand der Außenbereiche 12 zweite Vorsprünge 10 anzubringen. Diese zweiten Vorsprünge 10 würden dann beim Eingreifen der Klemmelemente 12 und 13 beim Nachlassen des Drucks auf die Außenbereiche 12 an den ersten Vorsprüngen 9 entlang gleiten.

Es ist auch denkbar, die ersten und zweiten zueinander komplementären Rastelemente der Führungsvorrichtung an den einander zugewandten Flächen der Klemmelemente 13 und 14 vorzusehen. Hierfür könnten beispielsweise an den Nasen der Widerhaken 13 und 14 entsprechende zusammenwirkende Vorsprünge vorgesehen sein. In diesem Fall wäre die Führungsvorrichtung und die Befestigungsvorrichtung in einer Vorrichtung vereint.

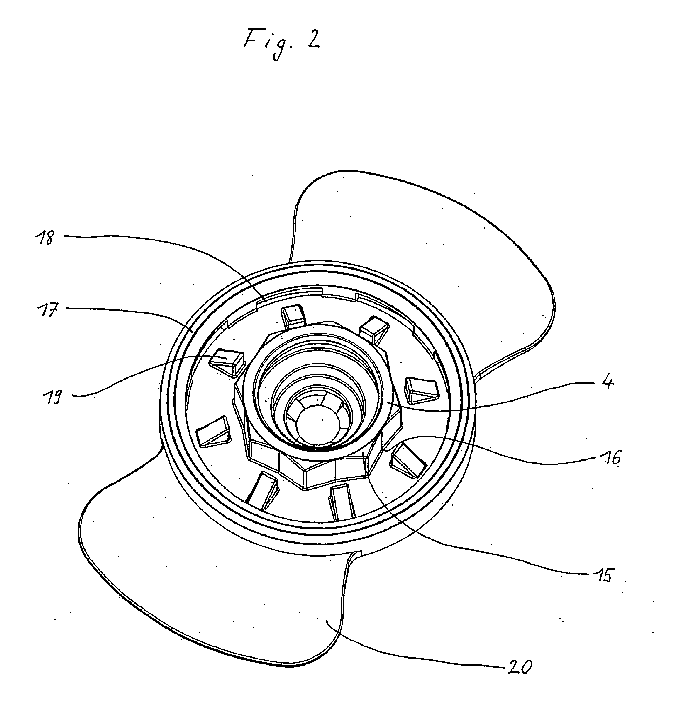

In Figur 2 ist ein Kanülengehäuse 1 eines anderen Ausführungsbeispiels eines erfindungsgemäßen Katheterkopfes gezeigt, der ebenfalls kreisförmig ausgebildet ist und von dem nach unten, in der Figur 2 nicht sichtbar, eine Kanüle hervorsteht. In dieser Ausführungsform ist die Führungsvorrichtung nach der Erfindung an der um die Mittelöffnung angeordneten Wand 4 vorgesehen. Zur Ausbildung einer Rastverbindung zwischen dem Kanülengehäuse 1 und einem Verbindungselement, um die diskreten Drehpositionen gemäß der Erfindung vorzusehen, sind an der Wand 4 Vorsprünge 15, vorzugsweise dreieckförmig, in Umfangsrichtung um die Wand vorgesehen. Durch diese dreieckförmigen Vorsprünge 15 wird der Außenumfang der Wand 4 in einer Art Sternform ausgebildet. Dabei ist es möglich, dass aneinander grenzende Wandflächen benachbarter Dreiecke in gleicher Richtung ausgerichtet sind und daher eine gemeinsame Fläche bilden oder, dass eine Vertiefung zwischen benachbarten Dreiecken entsteht, wenn die angrenzenden Flächen dieser Dreiecke in einem Winkel zueinander stehen. Ferner müssen die an der Wand vorgesehenen Vorsprünge 15 nicht dreieckförmig ausgebildet sein, beispielsweise können sie auch viereckförmig sein oder durch Rundungen gebildet werden.

Durch die an der Umfangsfläche der Wand 4 ausgebildeten Vorsprünge 15 werden die diskreten Drehpositionen dadurch vorgesehen, dass ein Zwischenraum 16 zwischen zwei Vorsprüngen 15, der zum Beispiel durch aneinander grenzende Wandflächen benachbarter dreieckförmiger Vorsprünge 15 gebildet wird, einer diskreten Drehposition entspricht. Durch die Variation der Anzahl der Vorsprünge an der Wand 4 kann die Anzahl der diskreten Drehpositionen eines Katheterkopfes gemäß der Erfindung variiert werden.

An einem zu dem Kanülengehäuse 1 einer Ausführungsform nach Figur 2 passenden Verbindungselement kann um den Fortsatz 8 eine Wand vorgesehen sein, die von dem Verbindungselement in Richtung des Kanülengehäuses 1 hervorragt. An einer Innenumfangsfläche dieser Wand können Vorsprünge vorgesehen werden, die zu den Vorsprüngen 15 des Kanülengehäuses 1 komplementär verlaufen. Für den Fall, dass von den Vorsprüngen 15 auf der Außenmfangsfläche der Wand 4 des Kanülengehäuses 1 Zwischenräume 16 gebildet werden, wie in Figur 2 gezeigt ist, sind die Vorsprünge auf der Innenumfangsfläche der Wand des Verbindungselements derart ausgebildet, dass sie in diese Zwischenräume 16 passen. Hierfür können beispielsweise dreieckförmige Vorsprünge mit einem stumpfen Winkel vorgesehen sein. Falls angrenzende Flächen benachbarter Vorsprünge 15 an der Wand 4 des Kanülengehäuses 1 eine gemeinsame Fläche bilden, ist die Innenumfangsfläche der Wand des Verbindungselements als Mehreck ausgebildet, dessen Innenflächen zu den gemeinsamen Flächen der Vorsprünge 15 passen. Sind die Vorsprünge 15 auf der Außenumfangsfläche der Wand 4 durch Rundungen gegeben, dann können auf der Innenumfangsfläche der Wand des Verbindungselements ebenfalls Rundungen ausgebildet sein, die in die Zwischenräume 16 passen.

Die Befestigungsvorrichtung für eine lösbare Klemmverbindung weist bei der Ausführungsform der Figur 2 an einem Außenbereich des Kanülengehäuses 1 eine ringförmig umlaufende Erhebung 17 auf. In der Innenumfangsfläche der Erhebung 17 sind mehrere langgestreckte in Umfangsrichtung verlaufende Vertiefungen 18 vorgesehen. An einem zu dieser Befestigungsvorrichtung passenden Verbindungselement können an den beweglichen Außenbereichen 12 Klemmelemente mit einem Vorsprung vorgesehen sein, der in die Vertiefungen 18 passt.

Das Auswählen einer diskreten Drehposition für den Katheterkopf und das Herstellen der Klemmverbindung zwischen dem Kanülengehäuse und dem Verbindungselement erfolgt analog der vorherigen in Figur 1 beschriebenen Ausführungsform. Um das Abnehmen des Verbindungselementes 2 durch das Zusammendrücken der Außenbereiche 12 und das Abheben vom Kanülengehäuse 1 zu erleichtern, sind vor den Vertiefungen 18 Führungsblöcke 19 auf der Grundfläche des Kanülengehäuses 1 für die Klemmelemente vorgesehen. Vorzugsweise sind die Vertiefungen 18 und die Zwischenräume 16 einander gegenüberliegend angeordnet und die Führungsblöcke 19 sind zwischen der Vertiefung 18 und den Zwischenräumen 16 angeordnet. Die Führungsblöcke 19 sind vorzugsweise als keilförmige Rampen ausgebildet, so dass sie eine Führungsfläche aufweisen, die vom Rand des Kanülengehäuses 1 in radialer Richtung ansteigt. Ein Klemmelement des Verbindungselements wird beim Abnehmen durch das Zusammenrücken der vorgespannten Außenbereiche 12 entlang dieser Führungsfläche nach oben von dem Kanülengehäuse abgestoßen.

Am Rand des Kanülengehäuses 1 sind auf gegenüberliegenden Seiten Laschen 20 vorgesehen, die zum Beispiel dem Halten des Kanülengehäuses 1 auf der Gewebeoberfläche beim Abheben des Verbindungselements dienen.

Die in den Zeichnungen gezeigten Ausführungsformen sind lediglich beispielhaft zu verstehen und sollen den Umfang der Erfindung nicht einschränken. Insbesondere kann die Winkelanordnung der Kanüle 3 und der Fluidzuführung 7 des erfindungsgemäßen Katheterkopfes verändert werden.

### Bezugszeichen

- 1: Kanülengehäuse
- 2: Verbindungselement
- 3: Kanüle
- 4: Wand
- 5: Septum
- 6: Durchgangskanal
- 7: Fluidzuführung
- 8: Fortsatz
- 9: erste Vorsprünge
- 10: zweite Vorsprünge
- 11: Öffnungsschlitze
- 12: Außenbereich
- 13: erstes Klemmelement
- 14: zweites Klemmelement
- 15: Wandvorsprung
- 16: Zwischenraum
- 17: Erhebung
- 18: Vertiefung
- 19: Führungsblock
- 20: Lasche

## Patentansprüche

1. Katheterkopf zur Einleitung eines Fluids in ein organisches Gewebe, wobei der Katheterkopf aufweist:
a) ein Kanülengehäuse (1) mit einer an einer Seite des Kanülengehäuses (1) hervorstehenden Kanüle (3) und einem durch das Kanülengehäuse (1) verlaufenden und mit der Kanüle (3) verbundenen Durchgangskanal (6),
b) ein Verbindungselement (2) mit einer Fluidzuführung (7), das mit dem Kanülengehäuse (1) derart verbindbar ist, dass die Fluidzuführung (7) mit dem Durchgangskanal (6) verbunden wird,
c) eine Führungsvorrichtung, die gemeinsam von dem Kanülengehäuse (1) und dem Verbindungselement (2) gebildet wird, zur Positionierung und Führung des Verbindungselements (2) relativ zu dem Kanülengehäuse (1), und
d) eine Befestigungsvorrichtung zur lösbaren Befestigung des Verbindungselements (2) an dem Kanülengehäuse (1),
**dadurch gekennzeichnet, dass**
e) die Führungsvorrichtung für die Positionierung des Verbindungselements (2) relativ zu dem Kanülengehäuse (1) um eine Längsachse (L) der Kanüle (3) mehrere wählbare diskrete Drehpositionen aufweist, und
d) die Befestigungsvorrichtung das in einer Drehposition positionierte Verbindungselement (2) in der ausgewählten Drehposition an dem Kanülengehäuse (1) lösbar befestigt.

2. Katheterkopf nach Anspruch 1, **dadurch gekennzeichnet, dass** die Fluidzuführung (7) nach dem Befestigen des Verbindungselements (2) an dem Kanülengehäuse (1) winkelig zur Längsachse (L) der Kanüle (3) angeordnet ist.

3. Katheterkopf nach Anspruch 1 oder 2, **dadurch gekennzeichnet, dass** die Kanüle (3) im wesentlichen normal von dem Kanülengehäuse (1) hervorsteht und die Fluidzuführung nach dem Befestigen im wesentlichen senkrecht zu der Kanüle (3) angeordnet ist.

4. Katheterkopf nach einem der vorhergehenden Ansprüche, **dadurch gekennzeichnet, dass** die diskrete Drehpositionen der Führungsvorrichtung frei wählbar sind.

5. Katheterkopf nach einem der vorhergehenden Ansprüche, **dadurch gekennzeichnet, dass** die Führungsvorrichtung für die wählbaren diskreten Drehpositionen eine Rastverbindung zwischen dem Kanülengehäuse (1) und dem Verbindungselement (2) bildet.

6. Katheterkopf nach dem vorhergehenden Anspruch, **dadurch gekennzeichnet, dass** die Rastverbindung gebildet wird durch:
- erste Rastmittel (9; 15), die zumindest auf einem Teilbereich einer Ringfläche des Kanülengehäuses (1) vorgesehen sind, und
- gegen die ersten Rastmittel (9; 15) gerichtete zweite Rastmittel (10), die zumindest auf einem Teilbereich einer Ringfläche des Verbindungselements (2), die dem Teilbereich der Ringfläche des Kanülengehäuses (1) zugewandt ist, vorgesehen sind und die beim Positionieren des Verbindungselements (2) mit den ersten Rastmitteln (9; 15) zusammenwirken.

7. Katheterkopf nach dem vorhergehenden Anspruch, **dadurch gekennzeichnet, dass** die ersten Rastmittel durch erste Vorsprünge (9; 15) und die zweiten Rastmittel durch zweite Vorsprünge (10) gebildet werden, wobei die zweiten Vorsprünge (10) an dem Verbindungselement (2) beim Positionieren des Verbindungselements (2) zwischen die ersten Vorsprünge (9; 15) an dem Kanülengehäuse (1) eingreifen.

8. Katheterkopf nach einem der vorhergehenden Ansprüche, **dadurch gekennzeichnet, dass** die Befestigungsvorrichtung eine lösbare Klemmverbindung zwischen dem Kanülengehäuse (1) und dem Verbindungselement (2) bildet.

9. Katheterkopf nach dem vorhergehenden Anspruch, **dadurch gekennzeichnet, dass** die Klemmverbindung gebildet wird durch:
- wenigstens einen länglichen Öffnungsschlitz (11) durch das Verbindungselement (2), durch den in dem Verbindungselement (2) wenigstens ein Außenbereich (12) gebildet wird, der in Richtung zu einem Zentrum des Verbindungselements (2) flexibel beweglich ist,
- wenigstens ein erstes Klemmelement (13), das an dem Kanülengehäuse (1) angeordnet ist, und
- wenigstens ein zweites Klemmelement (14), das an dem Außenbereich des Verbindungselements (2) an einer dem Kanülengehäuse (1) zugewandten Seite angeordnet ist und mit dem ersten Klemmelement (13) zusammenwirkt.

10. Katheterkopf nach einem der zwei vorhergehenden Ansprüche, **dadurch gekennzeichnet, dass** die Führungsvorrichtung durch ein Zusammenwirken von ersten und zweiten Klemmelementen (13, 14) der Befestigungsvorrichtung vorgesehen ist.

11. Katheterkopf nach einem der drei vorhergehenden Ansprüche, **dadurch gekennzeichnet, dass** die Rastverbindung der Führungsvorrichtung durch an den ersten und zweiten Klemmelementen (13, 14) vorgesehene und zusammenwirkende erste und zweite Rastmittel (9, 10; 15) gebildet wird.

12. Katheterkopf nach einem der vier vorhergehenden Ansprüche, **dadurch gekennzeichnet, dass** die Klemmverbindung gebildet wird durch:
- wenigstens einen länglichen Öffnungsschlitz (11) durch das Verbindungselement (2), durch den in dem Verbindungselement (2) wenigstens ein Außenbereich (12) gebildet wird, der in Richtung zu einem Zentrum des Verbindungselements beweglich ist,
- wenigstens ein erstes Klemmelement (13), das an dem Kanülengehäuse (1) angeordnet ist,
- zumindest einen ersten Widerhaken (13), der sich zumindest auf einem Teilbereich auf dem Kanülengehäuse (1) in Umfangsrichtung erstreckt und in Richtung des Verbindungselements (2) gerichtet ist, und
- zumindest einen zu dem ersten Widerhaken (13) komplementären zweiten Widerhaken (14), der sich zumindest auf einem Teilbereich an dem Außenbereich des Verbindungselements (2) in Umfangsrichtung erstreckt und in Richtung des Kanülengehäuses (1) gerichtet ist, und der zum Befestigen des Kanülengehäuses (1) mit dem Verbindungselement (2) in den ersten Widerhaken (13) eingreift.

13. Katheterkopf nach einem der vorhergehenden Ansprüche, **dadurch gekennzeichnet, dass** die Führungsvorrichtung zu einem Teil oder vollständig an der Befestigungsvorrichtung vorgesehen ist.

14. Katheterkopf nach einem der vorhergehenden Ansprüche, **dadurch gekennzeichnet, dass** die ersten und zweiten Rastmittel (9, 10; 15) beim Befestigen des Verbindungselement (2) mit dem Kanülengehäuse (1) aneinander gleiten.

15. Katheterkopf nach einem der vorhergehenden Ansprüche, **dadurch gekennzeichnet, dass** die ersten und zweiten Rastmittel (9, 10; 15) beim Eindrücken des wenigstens einen Außenbereichs des Verbindungselements (2) in der Richtung des Zentrums des Verbindungselements aneinander gleiten.

## Claims

1. A catheter head for introducing a fluid into an organic tissue, said catheter head comprising:
a) a cannula casing (1) comprising a cannula (3) protruding on one side of said cannula casing (1) and a passage channel (6) running through the cannula casing (1) and connected to said cannula (3);
b) a connecting element (2) which includes a fluid supply (7) and can be connected to the cannula casing (1) in such a way that said fluid supply (7) is connected to said passage channel (6);
c) a guiding means formed jointly by the cannula casing (1) and said connecting element (2), for positioning and guiding the connecting element (2) relative to the cannula casing (1); and
d) a fixing means for detachably fixing the connecting element (2) to the cannula casing (1);
**characterised in that**:
e) said guiding means for positioning the connecting element (2) relative to the cannula casing (1) exhibits a number of selectable, discrete rotational positions about the longitudinal axis (L) of the cannula (3); and
f) said fixing means detachably fixes the connecting element (2), positioned in a rotational position, to the cannula casing (1) in said selected rotational position.

2. The catheter head as set forth in claim 1, **characterised in that** once the connecting element (2) has been fixed to the cannula casing (1), the fluid supply (7) is arranged at an angle to said longitudinal axis (L) of the cannula (3).

3. The catheter head as set forth in claim 1 or 2, **characterised in that** the cannula (3) protrudes substantially perpendicularly from the cannula casing (1) and the fluid supply, once fixed, is arranged substantially perpendicular to the cannula (3).

4. The catheter head as set forth in any one of the preceding claims, **characterised in that** the discrete rotational positions of the guiding means can be freely selected.

5. The catheter head as set forth in any one of the preceding claims, **characterised in that** the guiding means forms a locking connection between the cannula casing (1) and the connecting element (2) for said selectable, discrete rotational positions.

6. The catheter head as set forth in the preceding claim, **characterised in that** said locking connection is formed by:
- first locking means (9; 15) which are provided at least on a partial region of an annular area of the cannula casing (1); and
- second locking means (10) directed against said first locking means (9; 15) which are provided at least on a partial region of an annular area of the connecting element (2) facing said partial region of said annular area of the cannula casing (1) and which co-operate with the first locking means (9; 15) when the connecting element (2) is positioned.

7. The catheter head as set forth in the preceding claim, **characterised in that** the first locking means are formed by first protrusions (9; 15) and the second locking means are formed by second protrusions (10), said second protrusions (10) on the connecting element (2) engaging between the first protrusions (9; 15) on the cannula casing (1) when the connecting element (2) is positioned.

8. The catheter head as set forth in any one of the preceding claims, **characterised in that** the fixing means forms a releasable clamping connection between the cannula casing (1) and the connecting element (2).

9. The catheter head as set forth in the preceding claim, **characterised in that** said clamping connection is formed by:
- at least one elongated opening slit (11) through the connecting element (2), by which at least one outer region (12) is formed in the connecting element (2) which can be flexibly moved towards a centre of the connecting element (2);
- at least one first clamping element (13) which is arranged on the cannula casing (1); and
- at least one second clamping element (14) which is arranged on said outer region of the connecting element (2) on a side facing the cannula casing (1) and co-operates with said first clamping element (13).

10. The catheter head as set forth in any one of the preceding two claims, **characterised in that** the guiding means is provided by first and second clamping elements (13, 14) of the fixing means co-operating.

11. The catheter head as set forth in any one of the preceding three claims, **characterised in that** the locking connection of the guiding means is formed by first and second locking means (9, 10; 15) which are provided on the first and second clamping elements (13, 14) and co-operate.

12. The catheter head as set forth in any one of the preceding four claims, **characterised in that** the clamping connection is formed by:
- at least one elongated opening slit (11) through the connecting element (2), by which at least one outer region (12) is formed in the connecting element (2) which can be moved towards a centre of the connecting element;
- at least one first clamping element (13) which is arranged on the cannula casing (1);
- at least one first barb (13) extending in the circumferential direction at least on a partial region on the cannula casing (1) and directed towards the connecting element (2); and
- at least one second barb (14), complementary to said first barb (13), which extends in the circumferential direction at least on a partial region on the outer region of the connecting element (2) and is directed towards the cannula casing (1), and which engages with the first barb (13) in order to fix the cannula casing (1) to the connecting element (2).

13. The catheter head as set forth in any one of the preceding claims, **characterised in that** the guiding means is provided partially or completely on the fixing means.

14. The catheter head as set forth in any one of the preceding claims, **characterised in that** the first and second locking means (9, 10; 15) slide on each other when the connecting element (2) is fixed to the cannula casing (1).

15. The catheter head as set forth in any one of the preceding claims, **characterised in that** the first and second locking means (9, 10; 15) slide on each other when said at least one outer region of the connecting element (2) is pressed in towards the centre of the connecting element.

## Revendications

1. Tête de cathéter pour introduire un fluide dans un tissu organique, ladite tête de cathéter comprenant:
a) un boîtier (1) porte-canule comportant une canule (3) saillant d'un côté dudit boîtier (1), et un canal traversant (6) parcourant ledit boîtier (1) et relié à ladite canule (3),
b) un élément de liaison (2) pourvu d'une amenée (7) de fluide, et pouvant être relié au boîtier (1) porte-canule de façon telle que ladite amenée (7) de fluide soit mise en liaison avec le canal traversant (6),
c) un dispositif de guidage conjointement matérialisé par le boîtier (1) porte-canule et par l'élément de liaison (2), en vue du positionnement et du guidage dudit élément de liaison (2) vis-à-vis dudit boîtier (1) porte-canule, et
d) un dispositif de fixation pour la fixation libérable de l'élément de liaison (2) sur le boîtier (1) porte-canule,
**caractérisée par le fait que**
e) le dispositif de guidage destiné au positionnement de l'élément de liaison (2) vis-à-vis du boîtier (1) porte-canule présente, autour d'un axe longitudinal (L) de la canule (3), plusieurs positions discrètes sélectionnables, prises par rotation ; et
f) dans la position sélectionnée prise par rotation, le dispositif de fixation provoque la fixation amovible de l'élément de liaison (2), placé dans une position prise par rotation, sur le boîtier (1) porte-canule.

2. Tête de cathéter selon la revendication 1, **caractérisée par le fait que** l'amenée (7) de fluide occupe une position angulaire vis-à-vis de l'axe longitudinal (L) de la canule (3) après que l'élément de liaison (2) a été fixé au boîtier (1) porte-canule.

3. Tête de cathéter selon la revendication 1 ou 2, **caractérisée par le fait que** la canule (3) dépasse pour l'essentiel perpendiculairement au-delà du boîtier (1) porte-canule et l'amenée de fluide occupe, à l'issue de la fixation, une position sensiblement perpendiculaire à ladite canule (3).

4. Tête de cathéter selon l'une des revendications précédentes, **caractérisée par le fait que** les positions discrètes, que le dispositif de guidage a prises par rotation, peuvent être sélectionnées librement.

5. Tête de cathéter selon l'une des revendications précédentes, **caractérisée par le fait que** le dispositif de guidage, affecté aux positions discrètes sélectionnables prises par rotation, matérialise une solidarisation par déclic entre le boîtier (1) porte-canule et l'élément de liaison (2).

6. Tête de cathéter selon la revendication précédente, **caractérisée par le fait que** la solidarisation par déclic est formée par :
- des premiers moyens d'encliquetage (9 ; 15), prévus au moins sur une région partielle d'une surface annulaire du boîtier (1) porte-canule, et
- des seconds moyens d'encliquetage (10) qui sont dirigés vers les premiers moyens d'encliquetage (9 ; 15) ; sont prévus au moins sur une région partielle d'une surface annulaire de l'élément de liaison (2) tournée vers la région partielle de la surface annulaire du boîtier (1) porte-canule ; et coopèrent avec lesdits premiers moyens d'encliquetage (9 ; 15) lors du positionnement dudit élément de liaison (2).

7. Tête de cathéter selon la revendication précédente, **caractérisée par le fait que** les premiers moyens d'encliquetage sont formés par des premières protubérances (9 ; 15) et les seconds moyens d'encliquetage sont formés par des secondes protubérances (10), les secondes protubérances (10) situées sur l'élément de liaison (2) s'engageant, lors du positionnement dudit élément de liaison (2), entre les premières protubérances (9 ; 15) situées sur le boîtier (1) porte-canule.

8. Tête de cathéter selon l'une des revendications précédentes, **caractérisée par le fait que** le dispositif de fixation matérialise une liaison amovible par coincement entre le boîtier (1) porte-canule et l'élément de liaison (2).

9. Tête de cathéter selon la revendication précédente, **caractérisée par le fait que** la liaison par coincement est formée par :
- au moins une fente d'ouverture allongée (11) traversant l'élément de liaison (2) et donnant naissance, dans ledit élément de liaison (2), à au moins une région extérieure (12) douée de mobilité flexible en direction d'un centre dudit élément de liaison (2),
- au moins un premier élément de coincement (13) disposé sur le boîtier (1) porte-canule, et
- au moins un second élément de coincement (14) qui est disposé sur la région extérieure de l'élément de liaison (2), d'un côté tourné vers le boîtier (1) porte-canule, et coopère avec ledit premier élément de coincement (13).

10. Tête de cathéter selon l'une des deux revendications précédentes, **caractérisée par le fait que** le dispositif de guidage est prévu par une coopération des premier et second éléments de coincement (13, 14) du dispositif de fixation.

11. Tête de cathéter selon l'une des trois revendications précédentes, **caractérisée par le fait que** la solidarisation par déclic du dispositif de guidage est matérialisée par des premiers et seconds moyens d'encliquetage (9, 10; 15) coopérant mutuellement, prévus sur les premier et second éléments de coincement (13, 14).

12. Tête de cathéter selon l'une des quatre revendications précédentes, **caractérisée par le fait que** la liaison par coincement est formée par :
- au moins une fente d'ouverture allongée (11) traversant l'élément de liaison (2) et donnant naissance, dans ledit élément de liaison (2), à au moins une région extérieure (12) douée de mobilité en direction d'un centre dudit élément de liaison (2),
- au moins un premier élément de coincement (13) disposé sur le boîtier (1) porte-canule,
- au moins un premier ardillon (13) qui s'étend périphériquement sur le boîtier (1) porte-canule, au moins sur une région partielle, et est orienté dans la direction de l'élément de liaison (2), et
- au moins un second ardillon (14) qui est complémentaire dudit premier ardillon (13) ; s'étend périphériquement sur la région extérieure de l'élément de liaison (2), au moins sur une région partielle ; est orienté dans la direction du boîtier (1) porte-canule ; et pénètre dans le premier ardillon (13) en vue de la fixation dudit boîtier (1) porte-canule et dudit élément de liaison (2).

13. Tête de cathéter selon l'une des revendications précédentes, **caractérisée par le fait que** le dispositif de guidage est prévu, pour partie ou intégralement, sur le dispositif de fixation.

14. Tête de cathéter selon l'une des revendications précédentes, **caractérisée par le fait que** les premiers et seconds moyens d'encliquetage (9, 10 ; 15) glissent les uns sur les autres lors de la fixation de l'élément de liaison (2) et du boîtier (1) porte-canule.

15. Tête de cathéter selon l'une des revendications précédentes, **caractérisée par le fait que** les premiers et seconds moyens d'encliquetage (9, 10 ; 15) glissent les uns sur les autres, dans la direction du centre de l'élément de liaison, lors de l'enfoncement de la région extérieure prévue au minimum sur ledit élément de liaison (2).
